# EUROPEAN PATENT APPLICATION

(11) **EP 1 132 084 A1**
(43) Date of publication of application: **12.09.2001**
(21) Application number: 00830169.9
(22) Date of filing: 06.03.2000
(51) Int. Cl.: A61K 31/35, A61K 9/70

(54) **Transdermal device for the slow release of soybean derived vegetable estrogens**

(71) Applicant: Docteur Nature Srl, 41041 Baggiovara (Modena) (IT)
(72) Inventor: Abram Bernard, 41041 Casinalbo di Formigine (Mo) (IT)
(74) Representative: Robba, Pierpaolo

(57) **Abstract**

A transdermal patch (2) for the administration, through the skin, of a phytotherapic composition, the patch comprising a back support film (4) and a matrix layer (6), applied onto the film (4) and containing a mixture of an anallergic acrylic glue and of soybean isoflavones.

## Description

The invention concerns a transdermal device, in particular a patch, for the administration of a phytotherapic composition.

The administration of active principles by means of transdermal devices, usually referred to as "patches", is nowadays a widely used method, since it is a particularly safe, reliable and well established technique.

Different types of transdermal patches are known, including e.g. the so-called "matrix patches", in which the active substance is mixed with a glue and is applied onto a suitable support membrane. When the patch is applied, the active substance slowly penetrates through the skin, passing from the glue layer to the organism's tissues.

Among the most common disorders arising in woman's organism and often giving rise to more serious diseases, disorders related with the occurrence of menopause are no doubt some of the most annoying. Actually, besides causing immediate effects, such as the well-known "skin rushes" or blushes, they cause a very significant increase in the risk of cardiovascular diseases or of osteoporosis.

In order to reduce such risks, a replacing hormonal therapy has proven necessary, just aiming at replacing the lack of production of estrogens by administering them from the outside. Such a therapy, which is nowadays a routine therapy for women during menopause, is generally based on synthetic hormones, which exhibit a good cost-to-benefit ratio. However, use of synthetic hormones, even though it improves the patient's quality of life and reduces the risks of the associated pathologies, causes an increase in other risk factors, such as cervix and breast cancer and water retention.

Recent clinical studies have however demonstrated the positive action of soybean isoflavones in treating disorders caused by menopause in woman's organism.

Isoflavones are active principles present in very low percentages in many foods, such as cereals and legumes. They act as the natural estrogens, by influencing the woman's genital apparatus especially when, during menopause, the physiological drop of estrogens production occurs.

Soybean isoflavones are therefore a natural remedy to the above problem, as it has been demonstrated by statistical studies carried out on far-east populations, the nourishment of which is based on soybean. Actually, soybean, among all phytoestrogen-containing foods, contains high levels of such phytoestrogens and indeed women in eastern countries, which make a massive use of soybean, do not suffer from problems related with menopause.

Isoflavones, which are obtained from a concentrate soybean extract, are commercially available in the form of capsules or tablets and are therefore administered by the oral route. Administration by the oral route, in the form of both tablets and syrups or infusions, has however some drawbacks: the active principle action is strongly dependent on the gastric absorption and, moreover, the active principles lose part of their effectiveness due to the attack by gastric juice.

The object of the invention is to provide a device for soybean isoflavone administration, which ensures a continuous and regular delivery, without suffering from the drawbacks of the conventional systems.

This object is attained by means of a transdermal device releasing soybean isoflavones.

A further object of the present invention is to use soybean isoflavones for preparing a transdermal patch for the treatment of menopause-related disorders:

The above and further objects of the invention are attained by the administering device made in accordance with the invention, as claimed in the appended claims.

Advantageously, the transdermal isoflavone-releasing device may be applied on a patient's skin, since it is anallergic, discrete and aesthetically acceptable.

The above and further objects of the invention will become more apparent from the description of a preferred embodiment, with reference to the accompanying drawings in which:
Fig. 1 shows a transdermal patch made in accordance with the invention, during a step of protecting film removal:
Fig. 2 is a top view of the transdermal patch of Fig. 1, after protecting film removal and
Fig. 3 is a cross-sectional view of the patch shown in Fig. 2.

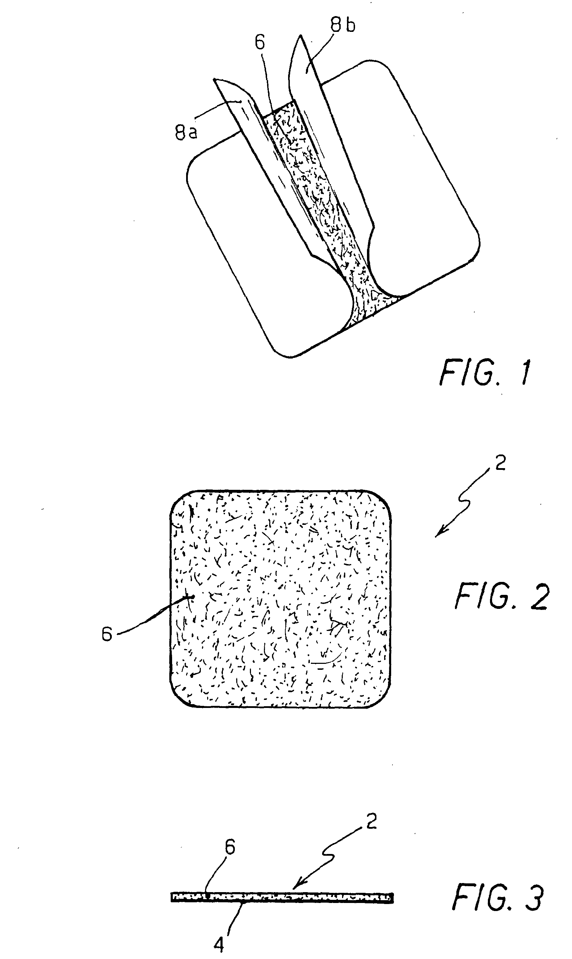

Referring to Figs. 1, 2 and 3, a transdermal patch 2 comprises a back support film 4, of substantially square shape, onto which a matrix layer 6 containing the active principle, in particular soybean isoflavones, is applied.

The back support film 4 is made of polyethylene and is adhesive on the side where the matrix layer 6 (glue plus active principle) is applied. Thus, the whole of the polyethylene support, up to the edge, is covered by the matrix that ensures the release of the active principle embedded therein, besides ensuring the complete and total adhesion to the skin.

In order to protect the patch, two removable films 8a, 8b are applied during production. Each film covers half the patch surface and has a raised end portion which assists in film removal before application. In the alternative, the front protection film could be a single film, provided e. g. with a margin projecting in correspondence with one of the patch corners.

Matrix layer 6, shown in cross-sectional view in Fig. 2, is composed by a mixture of a hypoallergenic acrylic glue, ensuring patch adhesion to the skin, and of an active principle embedded therein.

The active principle consists in soybean isoflavones, also known as phytoestrogens, which include genistine, genisteine, daidzine, daidzeine, glycitine and glyciteine.

Soybean isoflavones are obtained from soybean and introduced into the patch according to a process of extraction, mixing and spreading onto the support. The process comprises the following steps:
- extraction in anhydrous environment, with less than 3% water;
- extraction by means of a mixture of 80% ethyl alcohol and 20% USP propylene glycol;
- filtering on cellulose filter;
- definition of the certain isoflavone titration through the HPLC (High Pressure Liquid Chromatography) method;
- mixture with hypoallergenic acrylic glue;
- homogenisation at 18° to 25°;
- spreading on polyethylene support;
- total drying until complete solvent evaporation;
- on line control of weight and adhesives;
- wrapping up in class 100 (microbiologically controlled) environment;

HPLC method allows determining the isoflavone percentage in a soybean extract, said percentage forming the "title" of the extract and determining the therapeutic effectiveness thereof. The isoflavone title is the sum of the percentages of the individual isoflavones: genistine, genisteine, daidzine, daidzeine, glycitine and glyciteine.

Soybean isoflavones may be moreover associated with extract of Piper methysticum (kava-kava) which, in low dosage, has a tranquillising, antidepressant and psychorelaxing action. Actually, during menopause, the extrogen fall causes continuous mood changes, alterations of the sleeping-waking rhythm and hyperexcitability in women.

Indeed, deep studies have demonstrated the effectiveness of the kava-kava active principles, known as kavapirones, directly on the central nervous system.

The very limited size, 15 cm² only, the thickness, less than 0.2 mm, and the reduced weight, about 120 mg, make the present patch extremely comfortable, discrete and aesthetically acceptable.

Of course the patch shape is not binding: in the example a square-shaped patch has been disclosed, yet the patch could have any shape, e.g. a rectangular or circular shape.

The above patch, once applied onto the skin, releases, by passive diffusion, its active principles and allows an extremely comfortable, sure and effective treatment.

Moreover it is very easy to vary the dosage of the active principle in a patch, which normally is in the range 0.2 mg to 20 mg, during the manufacturing process, by varying the ratio of the glue to the active principle.

In the alternative, the user herself can intervene on the dosage, upon medical indication, by applying one or more patches, with the guarantee that the administration can be immediately stopped by removing the patch(es), should any problem arise.

Administration via the transdermal route has indeed innumerable advantages over other administration methods: for instance it is not so traumatic as an injection, both intravenous and intramuscular, and does not have the same contamination risks; moreover it is a system assisting in a lasting administration, with reduced doses.

The manufacture of the aforesaid anallergic patch, containing a natural active principle and devoid of any warning, contraindication or precaution for use, is an extremely valid innovation in the field of natural products, of paramount importance for all women suffering from the above problems.

## Claims

1. A transdermal patch (2) for the administration, through the skin, of a pharmaceutical composition, the patch being of a type comprising a back support film (4) onto which a layer (6) containing a glue mixed with said pharmaceutical composition is applied, **characterised in that** said pharmaceutical composition comprises soybean isoflavones.

2. A transdermal patch as claimed in claim 1, wherein said back support film (4) is a polyethylene film.

3. A transdermal patch as claimed in claim 1, wherein said back support film (4) is an adhesive film.

4. A transdermal patch as claimed in claim 1, further comprising at least one removable protective film (8a, 8b), arranged so as to cover said layer (6) and to be removed before applying said patch onto the skin.

5. A transdermal patch as claimed in claim 1, wherein said glue is a hypoallergenic acrylic glue.

6. A transdermal patch as claimed in claim 1, wherein said soybean isoflavones are homogeneously mixed with said glue, so as to obtain a uniform isoflavone distribution on the patch surface contacting the skin.

7. A transdermal patch as claimed in claim 1, wherein said isoflavones comprise at least one active principle out of genistine, genisteine, daidzine, daidzeine, glycitine and glyciteine.

8. A transdermal patch as claimed in claim 1, wherein the dosage of said soybean isoflavones in the layer (6) is in the range 0.2 mg to 20 mg.

9. A transdermal patch as claimed in claim 8, wherein the surface area of said layer (6) is of about 15 cm².

10. A transdermal patch as claimed in claim 1, wherein said pharmaceutical composition further includes kavapirones.

11. A transdermal patch as claimed in claim 1, wherein said kavapirones are contained in an extract of Piper methysticum.

12. Use of soybean isoflavones for the preparation of a transdermal patch (2) for the treatment of menopause-related disorders.
